# EUROPÄISCHE PATENTSCHRIFT

(11) **EP 1 899 878 B1**
(45) Veröffentlichungstag und Bekanntmachung des Hinweises auf die Patenterteilung: **10.04.2013**
(21) Anmeldenummer: 06753461.0
(22) Anmeldetag: 29.04.2006
(51) Int. Cl.: G06F 19/00, A61M 1/16

(54) **MEDIZINISCHES BEHANDLUNGSSYSTEM MIT EINER VORRICHTUNG ZUR BEREITSTELLUNG VON PATIENTENBEZOGENEN DATEN**
MEDICAL TREATMENT SYSTEM COMPRISING A DEVICE FOR PROVIDING PATIENT-RELATED DATA
SYSTEME DE TRAITEMENT MEDICAL COMPORTANT UN DISPOSITIF POUR PREPARER DES DONNEES SPECIFIQUES DE PATIENTS

(30) Priorität: 03.06.2005 DE 102005025516
(43) Veröffentlichungstag der Anmeldung: 19.03.2008
(60) Teilanmeldung: 11009143.6
(73) Patentinhaber: Fresenius Medical Care Deutschland GmbH, 61352 Bad Homburg (DE)
(72) Erfinder: BLÜMLER, Holger, 61352 Bad Homburg (DE); DANIEL, Pia, 78351 Bodmann (DE); ENDERS, Elmar, 97464 Niederwern (DE); GRÄFE, Marco, 61350 Bad Homburg (DE); GRÜNDKEN, Martin, 61191 Rosbach (DE); SCHUMACHER, Gerhard, 35510 Butzbach (DE); STAHL, Thomas, 97839 Esselbach (DE)
(74) Vertreter: Oppermann, Frank
(86) Internationale Anmeldenummer: PCT/EP2006/004049
(87) Internationale Veröffentlichungsnummer: WO 2006/128536

(56) Entgegenhaltungen:
- WO-A-01/65463
- WO-A2-02/093312
- DE-A1-102004 011 264
- US-B1- 6 188 407

## Beschreibung

Die Erfindung betrifft im Allgemeinen ein medizinisches Behandlungssystem mit mindestens einer Vorrichtung zur Durchführung einer medizinischen Behandlung und einer Vorrichtung zur Bereitstellung von patientenbezogenen Daten sowie im Speziellen ein extrakorporales Blutbehandlungssystem, insbesondere ein Dialysesystem mit mindestens einer Dialysevorrichtung und einer Vorrichtung zur Bereitstellung von patientenbezogenen Daten.

Aus der DE 10 2004 011 264 A1 ist eine Dialysestation bekannt, die eine Mehrzahl von Patientenplätzen und einen zentralen Server umfasst, so dass von einem Arztplatz aus nicht nur die Behandlung jedes Patienten überwacht, sondern auch in die Behandlung eingegriffen werden kann. Über das Datennetz ist ein Dialog zwischen dem Ar2tplatz und den einzelnen Patientenplätzen möglich. Weitere vernetzte Dialysesysteme sind aus der EP 1 195 708 A1 und der WO 01/37899 A2 bekannt.

Den bekannten Dialysesystemen ist gemeinsam, dass patientenbezogene Daten auf einem zentralen Server bereitgestellt werden, die von den einzelnen Behandlungsvorrichtungen geladen werden können. Darüber hinaus können patientenbezogene Daten, die in die Behandlungsvorrichtung eingegeben werden, auf dem zentralen Server gespeichert werden.

Aufgrund der erhöhten Sicherheitsanforderungen in der Medizintechnik werden an den Datentransfer zwischen den Behandlungsvorrichtungen und dem Server sehr hohe sicherheitstechnische Anforderungen gestellt, um zum Einen Fehlbedienungen und zum Anderen Fehlfunktionen des medizinischen Behandlungssystems ausschließen zu können.

Die WO 01/65463 beschreibt ein medizinisches Behandlungssystem, das über Vorrichtung zur Durchführung medizinischer Behandlungen und eine Vorrichtung zur Bereitstellung von patientenbezogenen Daten verfügt. Zwischen den Behandlungsvorrichtungen und der Vorrichtung zur Bereitstellung von patientenbezogenen Daten findet eine bidirektionale Kommunikation statt. Die Behandlungsvorrichtungen und die Vorrichtung zur Bereitstellung von patientenbezogenen Daten verfügen über Einrichtungen zur Eingabe und Anzeige von sowohl patientenbezogenen als auch maschinenbezogenen Daten.

Aus der US 6 188 407 B1 ist eine Anzeigeeinrichtung für eine medizinische Behandlungsvorrichtung bekannt, die über einen Bildschirm verfügt, auf dem in einem Anzeigebereich innerhalb vorgegebener Grenzen patientenbezogene Datensätze dargestellt werden. Die patientenbezogenen Datensätze werden in Form von vorgegebenen Symbolen (vital signs) dargestellt, die in ihrer Größe variieren können. Für den Fall, dass auf dem Anzeigebereich genügend Platz zur Darstellung sämtlicher Symbole, die dargestellt werden sollen, zur Verfügung steht, werden die Symbole in einem großen Format angezeigt, während Symbole in einem kleinen Format dargestellt werden, wenn für die Darstellung sämtlicher Symbole nicht genügend Platz auf dem Anzeigebereich zur Verfügung steht. Auf jeden Fall soll das System sicherstellen, dass immer alle Symbole dargestellt werden, die angezeigt werden sollen.

Der Erfindung liegt die Aufgabe zugrunde, ein medizinisches Behandlungssystem, insbesondere ein Dialysesystem anzugeben, das eine umfassende Unterrichtung der Bedienperson über die medizinische Behandlung erlaubt, wobei Fehleingaben und Fehlfunktionen mit hoher Sicherheit ausgeschlossen sind.

Die Lösung dieser Aufgabe erfolgt erfindungsgemäß mit den Merkmalen des Patentanspruchs 1.

Die Eingabe- / Anzeigeeinrichtung des medizinischen Behandlungssystems ist derart ausgebildet, dass in einem Behandlungsmodus maschinenbezogene Datensätze und in einem Patientenmodus patientenbezogene Datensätze angezeigt werden. Dabei werden unter maschinenbezogenen Datensätze alle Daten verstanden, die sich auf die Maschinensteuerung beziehen, beispielsweise die Förderraten der Blut- und Dialysierflüssigkeitspumpe, die Ultrafiltrationsrate etc., während unter patientenbezogenen Datensätze alle Daten verstanden werden, die auf die Steuerung der Behandlungsvorrichtung keinen Einfluss haben. Diese Datensätze dienen ausschließlich der Information der Bedienperson.

Die Eingabe- / Anzeigeeinrichtung ist als Touch-Screen zur Eingabe und Anzeige der patientenbezogenen Daten ausgebildet, wobei die Mittel zur Eingabe und Anzeige der unterschiedlichen Gruppen von patientenbezogenen Daten auf dem Bildschirm darstellbare Seiten mit einzeln selektierbaren Feldern sind.

Der Datentransfer findet zwischen der Behandlungsvorrichtung und einer Vorrichtung zur Bereitstellung von patientenbezogenen Daten statt, die eine Einrichtung zur Speicherung der patientenbezogenen Daten aufweist.

In dem Patientenmodus ist ein patientenbezogener Anzeigebereich nur zur Anzeige von patientenbezogenen Datensätzen definiert, der ausschließlich der Information des Bedienpersonal über den Patienten, nicht aber die Steuerung der Behandlungsvorrichtung dient. Der patientenbezogene Anzeigebereich ist ein Teil des als Touch-Screen ausgebildeten Bildschirms der Behandlungsvorrichtung.

Bei der Übertragung der Daten von der Vorrichtung zur Bereitstellung der patientenbezogenen Daten auf die einzelnen Behandlungsvorrichtungen besteht die Gefahr, dass sich die Datensätze nicht vollständig darstellen lassen. Zur Vermeidung von Fehlbedienungen bzw. Fehlinformationen verfügt die Eingabe- / Anzeigeeinrichtung über Mittel zum Überprüfen, ob die Darstellung eines patientenbezogenen Datensatzes innerhalb der Grenzen des patientenbezogenen Anzeigebereichs ohne Überlappung mit einem bereits dargestellten Datensatz erfolgen kann, wobei die Anzeige der Datensätze bei einer Überschreitung der Grenzen oder einer Überlappung verhindert wird. Dadurch wird sichergestellt, dass der Bedienperson nicht nur ein Teil der Informationen zur Verfügung stehen kann, was zu fehlerhaften Rückschlüssen fuhren könnte.

Im Folgenden wird ein Ausführungsbeispiel der Erfindung unter Bezugnahme auf die Zeichnungen näher erläutert.

Es zeigen:
- Fig. 1: ein extrakorporales Blutbehandlungssystem in vereinfachter schematischer Darstellung,
- Fig. 2: die Bildschirmoberfläche der Behandlungsvorrichtung im Behandlungsmodus,
- Fig. 3: die Bildschirmoberfläche der Behandlungsvorrichtung im Patientenmodus,
- Figuren 4A bis 4D: den zentralen Anzeigebereich der Bildschirmoberfläche im Patientenmodus, wobei die Ereignis-Seite aufgerufen ist,
- Figuren 5A bis 5C: ein Flussdiagramm zur Erläuterung des logischen Ablaufs der Bedienung nach dem Aufruf der Medikamenten-Seite und
- Figuren 6A und 6B: ein Flussdiagramm zur Erläuterung des logischen Ablaufs der Bedienung nach dem Aufruf der Nachrichten-Seite.

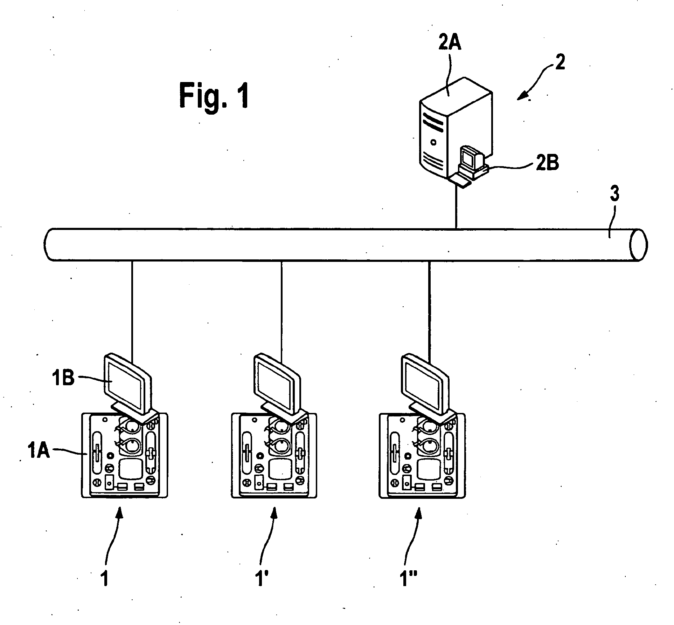

Figur 1 zeigt die wesentlichen Komponenten eines extrakorporalen Blutbehandlungssystems, insbesondere eines Hämodialysesystems, in stark vereinfachter schematischer Darstellung. Das Dialysesystem umfasst eine Mehrzahl von Dialysevorrichtungen 1, 1', 1" und eine Vorrichtung 2 zur Bereitstellung von patientenbezogenen Daten, die über ein Netzwerk 3 miteinander verbunden sind.

Die Vorrichtung zur Bereitstellung der patientenbezogenen Daten 2 (Server) verfügt über eine Einrichtung 2A zum Speichern der patientenbezogenen Daten, beispielsweise einen Festplattenspeicher, und eine Einrichtung 2B zur Eingabe der patientenbezogenen Daten. Beispielsweise kann die Vorrichtung einen PC mit Bildschirm und Tastatur umfassen.

Die einzelnen Dialysevorrichtungen 1, 1', 1" verfügen jeweils über einen Maschinenteil 1A und eine Eingabe- / Anzeigeeinrichtung 1B. Bei der Eingabe- /Anzeigeeinrichtung handelt es sich um einen als Touch-Screen ausgebildeten Bildschirm, mit dem sowohl maschinenbezogene Daten zur Steuerung der Dialysevorrichtung als auch patientenbezogene Daten eingegeben und angezeigt werden können. Der Maschinenteil 1A der Dialysevorrichtung weist eine zentrale Steuereinheit zur Steuerung der einzelnen Maschinenkomponenten auf.

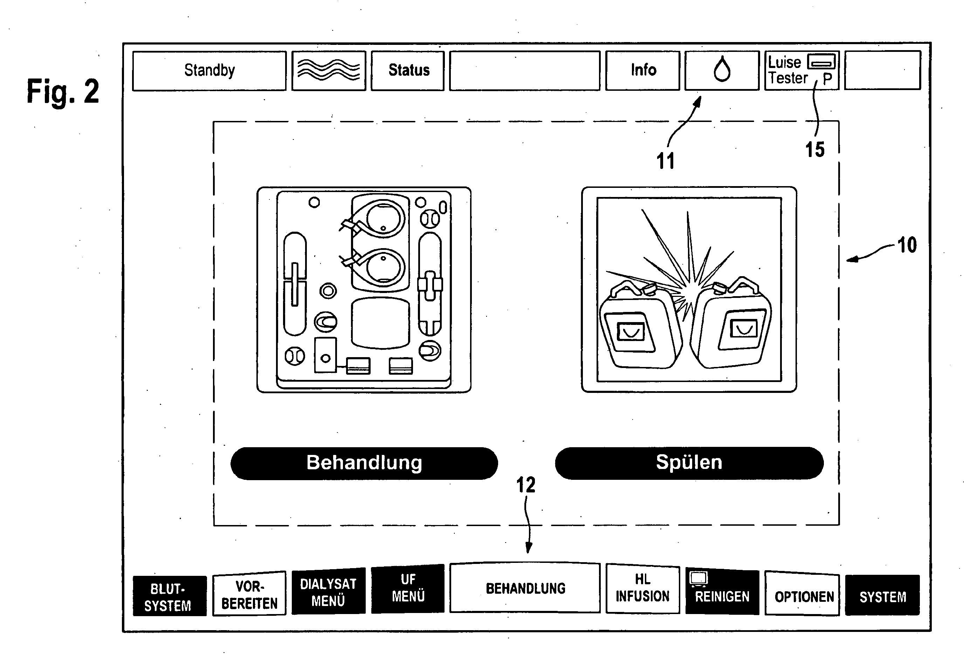

Figur 2 zeigt die Bildschirmoberfläche des Bildschirms 1A der Dialysevorrichtung 1. Die Bildschirmoberfläche gliedert sich in einen zentralen Anzeigebereich 10 und eine Status-Leiste 11 am oberen Rand und eine Menü-Leiste 12 am unteren Rand des Bildschirms. Die Grenzen des zentralen Anzeigebereichs 10 sind in Fig. 2 gestrichelt dargestellt. Die Status-Leiste 11 und die Menü-Leiste 12 weisen jeweils eine Mehrzahl von selektierbaren Feldern (Button) auf, die mit dem Finger, aber auch mit einem Cursor ausgewählt werden können.

Die Bedienperson kann auf dem Bildschirm zwischen einem Behandlungsmodus und einem Patientenmodus wählen. Figur 2 zeigt den Behandlungsmodus, in dem maschinenbezogene Daten eingegeben und angezeigt werden können. In diesem Modus können beispielsweise die für die Behandlung vorgegebenen Parameter für die zentrale Steuereinheit der Dialysevorrichtung eingegeben werden.

Der Patientenmodus, in dem patientenbezogene Daten auf dem Bildschirm eingegeben und angezeigt werden können, stellt eine von dem Behandlungsmodus völlig unabhängige Funktion dar. Eingaben und Anzeigen im Patientenmodus haben somit keinen Einfluss auf die Dialysebehandlung. Sie dienen ausschließlich der Information und Dokumentation.

Die Eingabe und Anzeige von patientenbezogenen Daten im Patientenmodus erfolgt innerhalb des zentralen Anzeigebereichs 10, so dass die Statusleiste 11 und die Menüleiste 12 sowie die linken und rechten Anzeigebereiche 13, 14 sichtbar bleiben, in denen weitere maschinenbezogene Daten, beispielsweise der arterielle und venöse Blutdruck sowie der Blutfluss dargestellt werden können.

Die patientenbezogenen Daten werden vom Server 2 des Dialysesystems geladen. Da die Daten zentral bereitgestellt werden, sind Änderungen und Anpassungen an Kundenwünsche kurzfristig möglich. Im Patientenmodus können dynamische, interaktive Programmsequenzen ablaufen, die in der Dialysevorrichtung nicht gespeichert sind, sondern vom Server geladen werden. Da die Programmsequenzen in der Dialysevorrichtung isoliert von der Maschinensteuerung ablaufen, können sie die medizinische Funktion der Dialysevorrichtung nicht beeinflussen.

Die interaktiven Programmsequenzen dienen einerseits der Darstellung der patientenbezogenen Daten auf dem Bildschirm im Patientenmodus, beispielsweise Labordaten. Darüber hinaus sind Eingaben der Bedienperson möglich. Als Reaktion auf die Eingaben kann der Server weitere Daten an die Dialysevorrichtung senden und somit eine gezielte Benutzerführung ermöglichen.

Die Interaktivität der Programmsequenzen wird über dynamisch vom Server geladene Bildschirmseiten erreicht, deren Aufbau einer Seitenbeschreibungssprache folgt. Die Kommunikation zwischen der Dialysevorrichtung und dem Server erfolgt mit dem Protokoll http bzw. HTTPS, wobei der Inhalt der Seiten in HTML beschrieben wird. In den Seitenbesehreibungen sind Informationen darüber enthalten, welche Bedienelemente auf dem Bildschirm darzustellen sind, welche Attribute sie besitzen, wo sie platziert sind und wie sie auf Benutzereingaben reagieren sollen.

Die einfachste Form einer Programmsequenz ist die Darstellung einer vom Server generierten Bildschirmseiten mit keinen oder sehr wenigen Möglichkeiten zur Dateneingabe durch die Bedienperson. Diese Seite wird beispielsweise über einen Menüpunkt angewählt und über eine Schaltfläche geschlossen.

Eine komplexe Programmsequenz kann eine Sequenz von Bildschirmseiten beinhalten, deren Reihenfolge und Inhalt abhängig von Eingaben des Bedienpersonals festgelegt wird. Ein Beispiel hierfür sind interaktive Hilfe- oder Trainingsseiten, deren Ablauf über die Schaltflächen gesteuert wird.

Eine noch komplexere Programmsequenz ermöglicht es, patientenbezogene Dateneingaben entgegenzunehmen, im Server unter Zuhilfenahme von Daten, die anderweitig zur Verfügung gestellt werden, beispielsweise aus einer Dialysedatenbank oder durch Messdatenakquisition aus dem Dialysegerät selbst, um einen Rechenalgorithmus auszuführen und damit medizinisch relevante Parameter zu berechnen, die für die aktuelle oder kommende Behandlungen des Patienten relevant sein können.

Nachfolgend wird die Funktion des Bildschirms im Patientenmodus anhand von Beispielen näher erläutert.

Die Dialysevorrichtung fragt in vorgegebenen Zeitabständen zyklisch beim Server an, ob neue patientenbezogene Daten vorliegen. Wenn dies der Fall ist, blinkt ein Patienten-Button 15, der den Namen des Patienten trägt, in der Statusleiste 11 des Bildschirms, sofern der Behandlungsmodus, nicht aber der Patientenmodus aktiviert ist. Erst wenn der Patienten-Button 15 angewählt wird, öffnet sich der Patientenmodus. Es ist in jedem Fall sichergestellt, dass die Bedienperson immer über alles sicherheitsrelevanten Daten informiert ist.

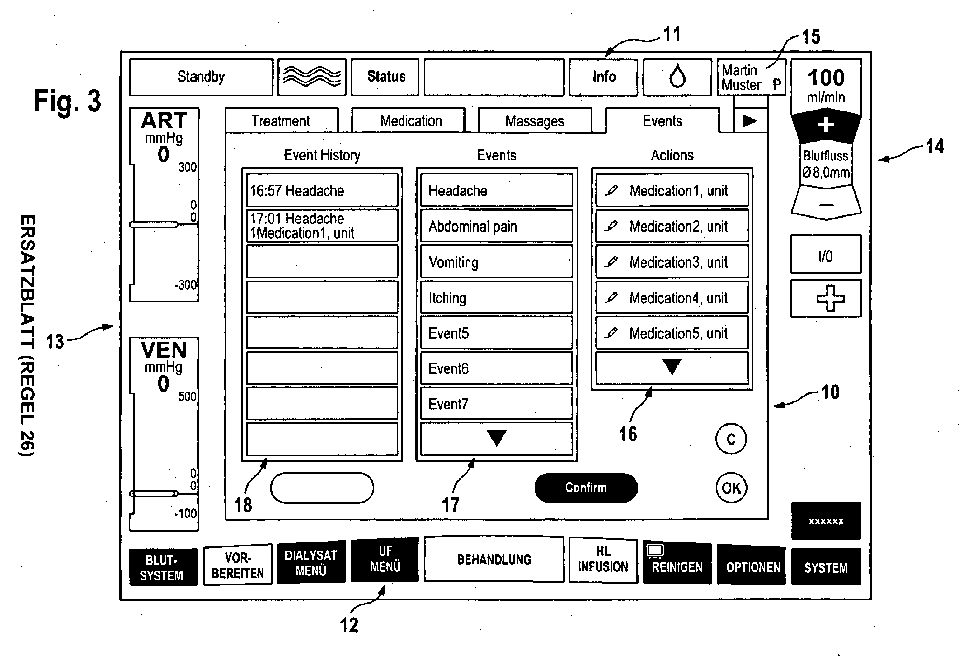

Die patientenbezogenen Daten definieren grafische Elemente, die auf dem Bildschirm dargestellt werden können. Figur 3 zeigt die Bildschirmoberfläche im Patientenmodus. Die grafischen Elemente können von dem Server frei innerhalb des zentralen Anzeigebereichs 10 (Fig. 2) platziert werden. Bevor die patientenbezogenen Daten auf dem Bildschirm im Patientenmodus zur Anzeige gebracht werden, wird überprüft, ob eine Anzeige der einzelnen grafischen Elemente, innerhalb der vorgegebenen Grenzen des zentralen Anzeigebereichs 10 ohne Überlappung mit bereits dargestellten Elementen möglich ist. Wenn dies der Fall ist, wird die Anzeige der Elemente verhindert. Ansonsten werden die Elemente im zentralen Anzeigebereich des Bildschirms angezeigt. Dadurch wird sichergestellt, dass nur dann eine Anzeige erfolgt, wenn sämtliche relevanten Informationen zur Verfügung stehen, so dass Fehlinterpretationen der dargestellten Inhalte ausgeschlossen sind.

Die grafischen Elemente werden gemäß den Positionsangaben in der HTML-Seitenbeschreibung zunächst virtuell platziert. Dabei wird bei jedem Element überprüft, ob der Platz nicht schon für ein anderes Element reserviert ist und das Element vollständig innerhalb des zentralen Anzeigebereichs liegt. Erst dann wird der Platz für dieses Element freigegeben. Sollte der Platz aber schon belegt sein, wird die Seite nicht dargestellt und eine Fehlermeldung auf der Seite angezeigt. Dabei bleibt der übrige Teil des Bildschirms bedienbar. Erst wenn alle Elemente erfolgreich ohne Kollision virtuell platziert werden konnten, wird die komplette Seite zur Anzeige gebracht.

Zur Vermeidung von Fehlfunktionen werden auf dem Bildschirm nur die grafischen Elemente dargestellt, die den Dialysevorrichtungen bekannt sind. Dazu werden die HTML-Elemente mit einem sogenannten class-Attribut versehen. Vor der Darstellung der einzelnen Elemente auf dem Bildschirm wird geprüft, ob die Elemente mit einem class-Attribut versehen sind, wobei nur die Elemente dargestellt werden, die mit einem class-Attribut versehen sind. Elemente ohne class-Attribut hingegen werden ignoriert. Für den Fall, dass der Server über eine neuere Softwareversion als eine der Dialysevorrichtungen verfügen sollte, könnten Elemente mit unbekannten class-Attributen zur Verfügung stehen. Diese Elemente mit unbekannten class-Attributen werden nicht dargestellt, so dass Fehlfunktionen ausgeschlossen sind. Allerdings erhält der Benutzer einen Hinweis.

Im Patientenmodus werden unterschiedliche Gruppen von patientenbezogenen Daten auf unterschiedlichen Seiten angezeigt. Zu den unterschiedlichen Gruppen zählen beispielsweise die durchgeführte Behandlung, die verordneten Medikamente, Nachrichten, Ereignisse oder Aktionen. Die einzelnen Seiten können mit dem Finger durch Betätigung des entsprechenden Button am oberen Rand der Seiten aufgerufen werden. In Fig. 3 ist die Ereignis-Seite dargestellt. Jede Seite enthält eine Mehrzahl von Datensätzen, zu denen bei der Ereignis-Seite beispielsweise die verordneten Medikamente, beispielsweise Medikament 1, Medikament 2, Medikament 3 ... , oder unterschiedliche Ereignisse, beispielsweise Kopfschmerzen ... , zählen. Diese Datensätze sind in einzelnen Listen 16, 17, 18 gruppiert. Wenn während der Behandlung ein Ereignis auftritt, kann das Ereignis mit Uhrzeit und einer entsprechenden Aktion dokumentiert werden. Hierzu wird der jeweilige Button angewählt.

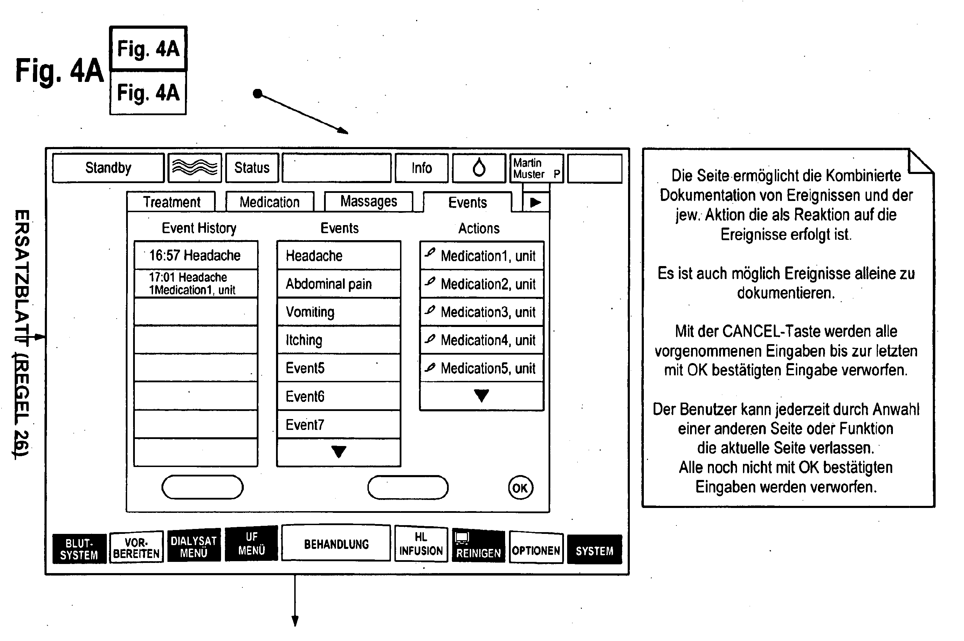

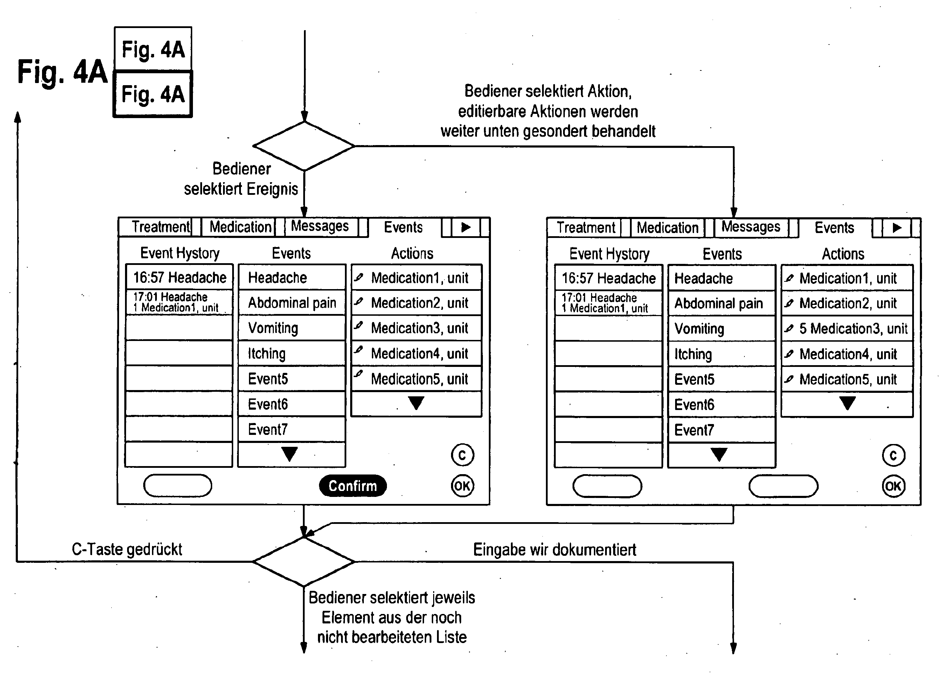

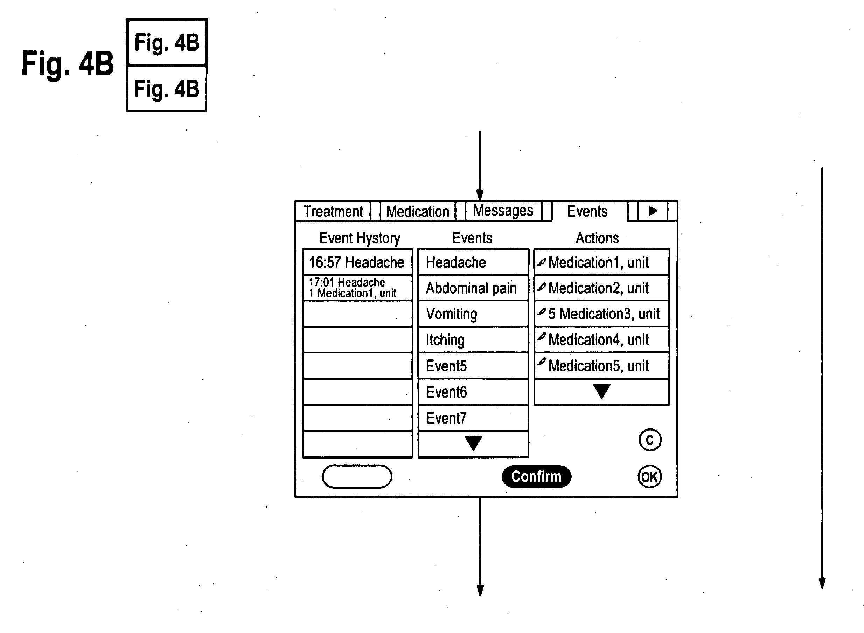

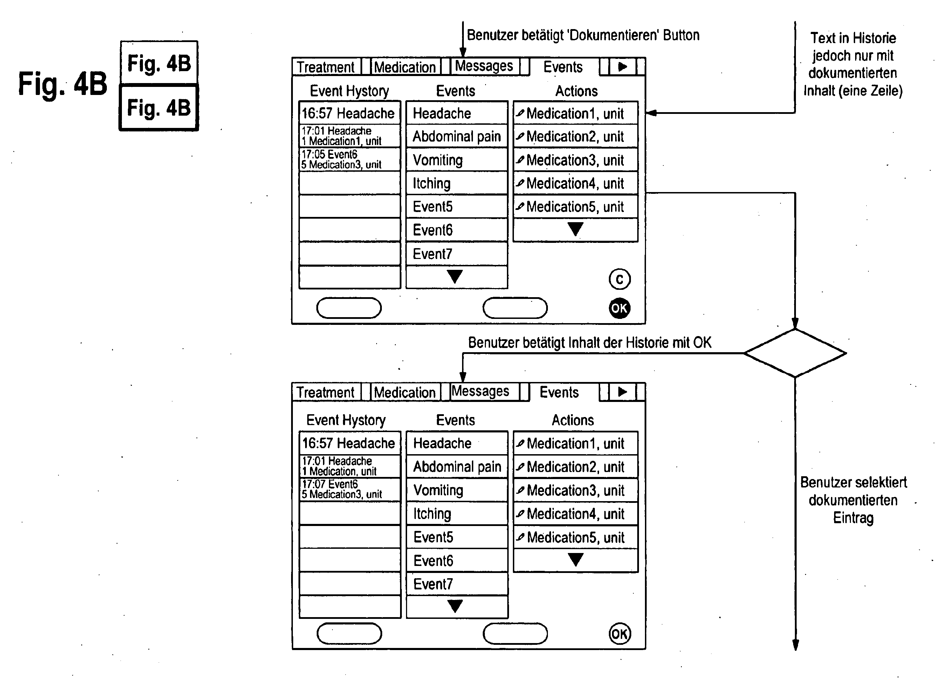

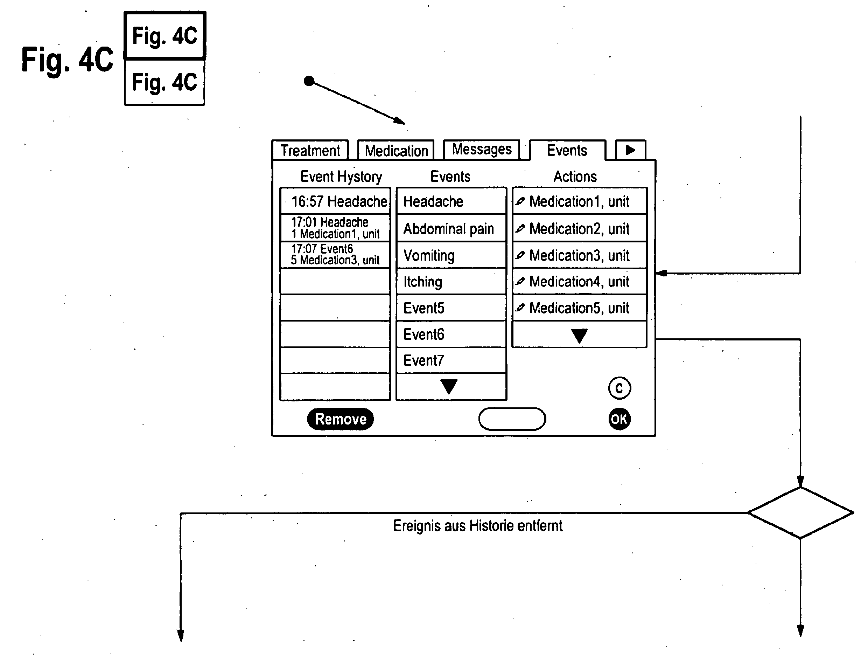

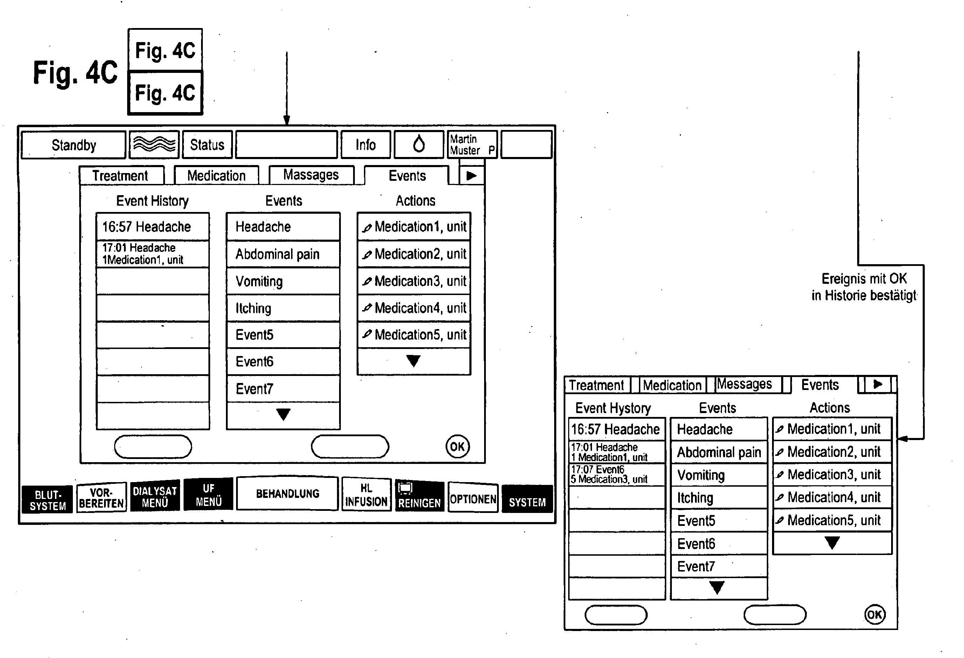

Im Folgenden wird unter Bezugnahme auf die Figuren 4A bis 4D die Menüauswahl für die Anzeige und Dokumentation von Ereignissen und Aktionen näher erläutert. Die Bedienperson wählt beispielsweise in der Ereignis-Liste 17 unter den verschiedenen vorgegebenen Ereignissen "Kopfschmerzen ... " das Ereignis "Ereignis 6" aus. Dadurch wird dieser Schriftzug grün unterlegt. Anstelle eines Ereignisses kann die Bedienperson auch zunächst eine Aktion auswählen. Wenn die Bedienperson zunächst ein Ereignis ausgewählt hat, kann nunmehr die Auswahl einer Aktion erfolgen.

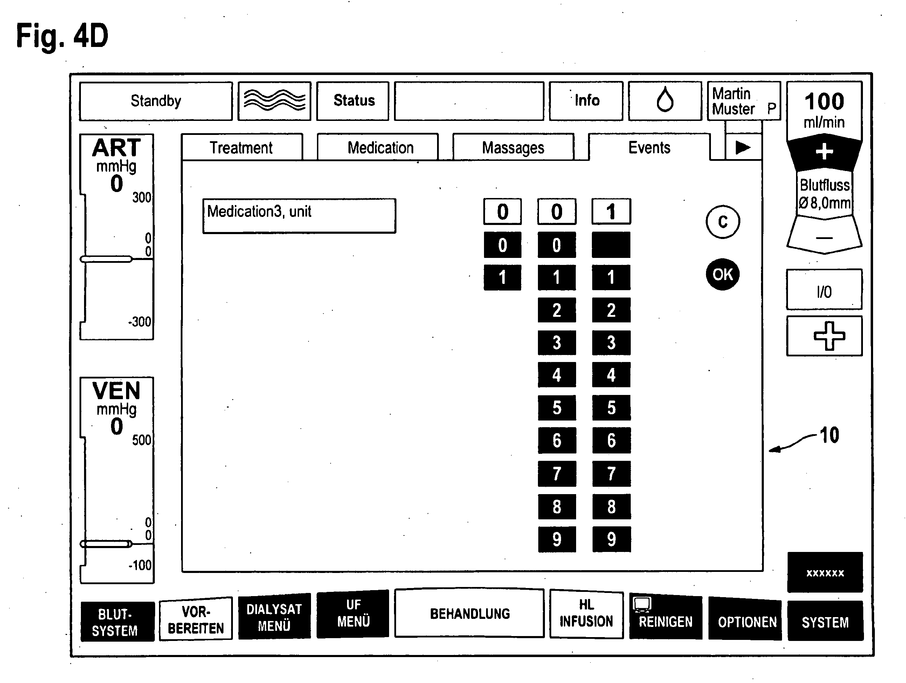

Die Bedienperson wählt beispielsweise aus der Aktions-Liste 16 die Aktion "Medikamtent2, Einheit" aus. Damit wird die Verabreichung eines bestimmten Medikaments aus der Liste der Medikamente ausgewählt. Dann öffnet die Bedienperson eine neue Seite (Fig. 4D), auf der die Anzahl der verabreichten Einheiten eingeben wird. Beispielsweise gibt die Bedienperson durch Betätigung des entsprechenden Button 5 Einheiten ein. Daraufhin kehrt die Bedienperson wieder zu der vorhergehenden Seite zurück. Die Aktion "5 Medikament 3, Einheit" ist jetzt grün unterlegt. Dem ausgewählten Datensatz "Medikament 3, Einheit" ist der eingegebene Datensatz "5" zugeordnet worden.

Nach Bestätigung der Eingaben werden die ausgewählten Ereignisse und Aktionen in einer Liste 18 von abgespeicherten Ereignissen (Event History) unter Angabe des Zeitpunktes der Eingabe (17:05) angezeigt. Der Schriftzug ist blau hinterlegt. Diese Anzeige kann der Benutzer durch Betätigung des Button "OK" bestätigen. Der Schriftzug ist nun grau unterlegt. Es besteht aber auch die Möglichkeit, die in der "Event History" angezeigten Ereignisse und Aktionen aus der "History" zu entfernen. Hierzu betätigt der Benutzer den Button "Entfernen".

Der Patientenmodus erlaubt also die Zuordnung von bestimmten auszuwählenden Ereignissen und bestimmten auszuwählenden Aktionen, die zur Dokumentation der Reihe nach abgespeichert und angezeigt werden können. Dabei erfolgt die Speicherung der Datensätze in dem Server 2 unabhängig von den einzelnen Dialysevorrichtungen 1, 1', 1". Der Benutzer wird also vor der Auswahl neuer Ereignisse und Aktionen über die bereits erfolgten Ereignisse und Aktionen unterrichtet. Beispielsweise wird dem Benutzer vor der Eingabe des Ereignisses "Event 6" und der Aktion "5 Medikament 3, Einheit" angezeigt, dass der Patient bereits zuvor Kopfschmerzen hatte und ein Medikament verabreicht worden ist. Die Verknüpfung der einzelnen Datensätze, zwischen denen der Benutzer auswählen kann, sowie die Dokumentation der Datensätze erhöht die Sicherheit bei der Dialysebehandlung.

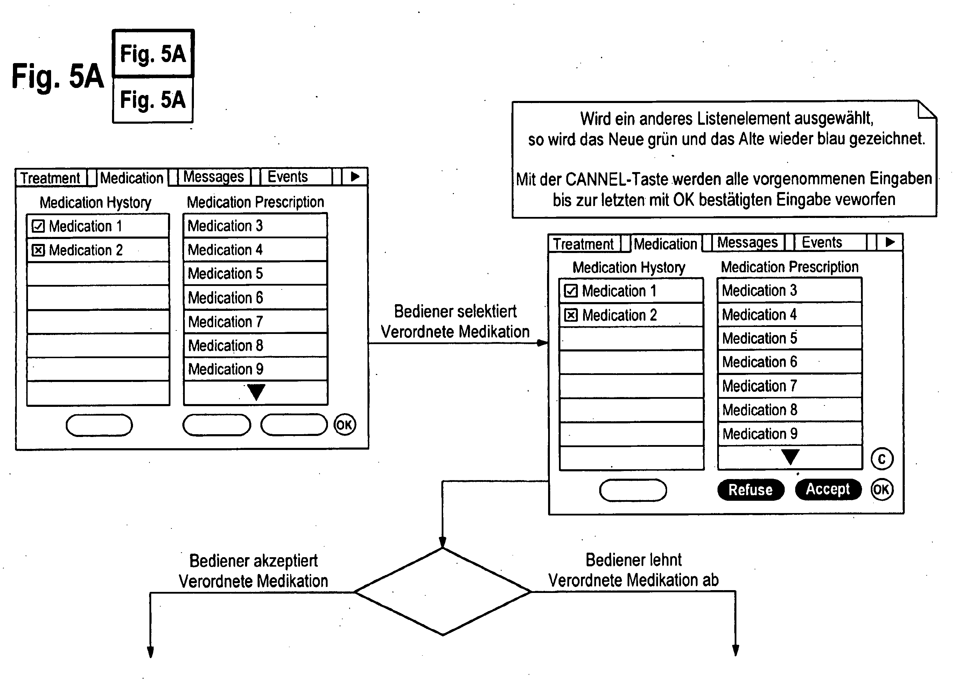

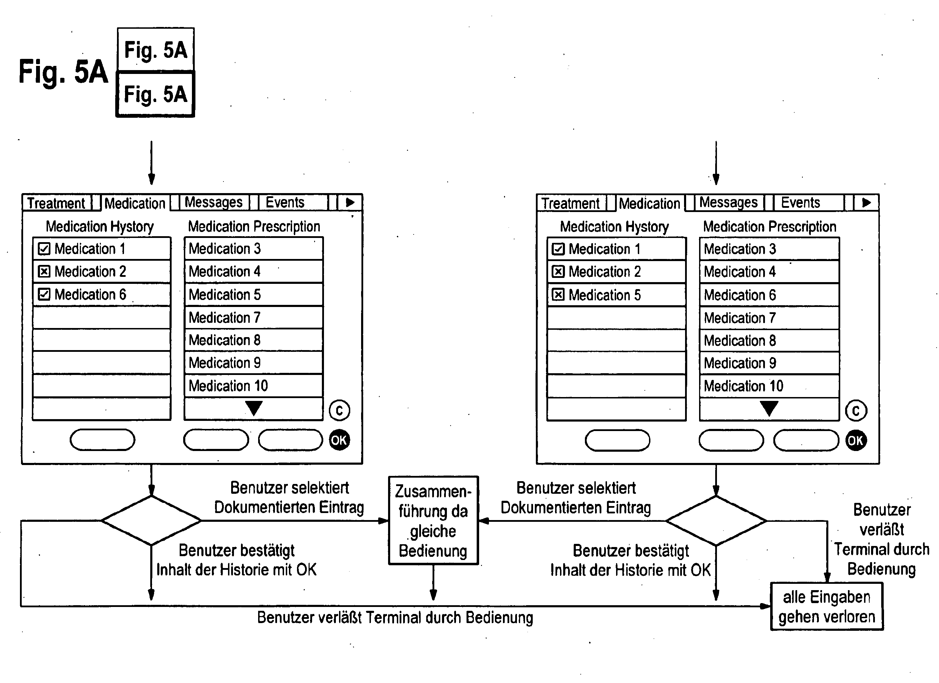

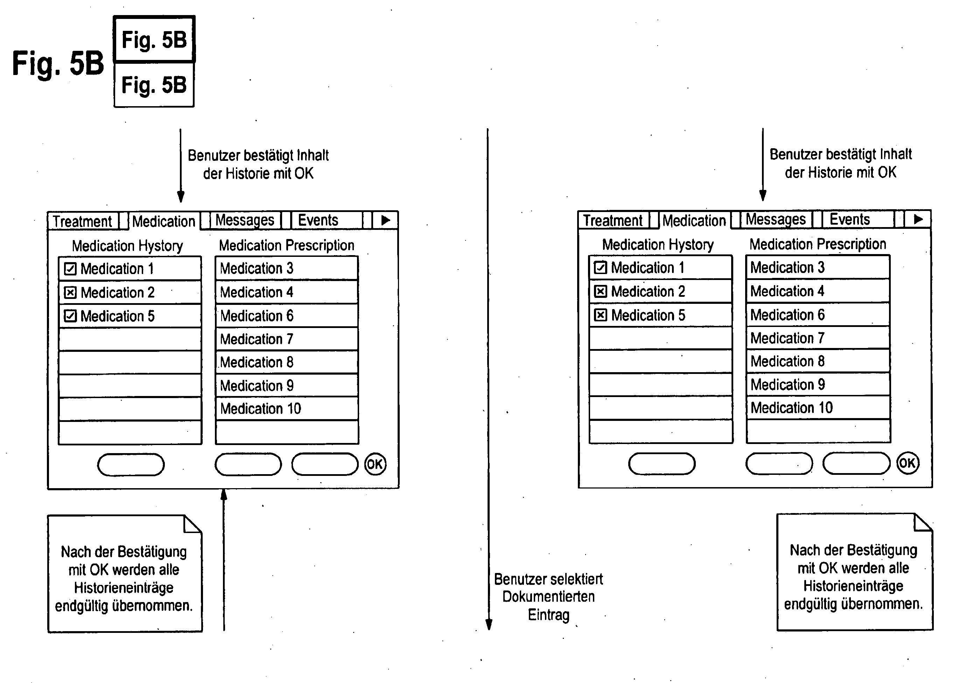

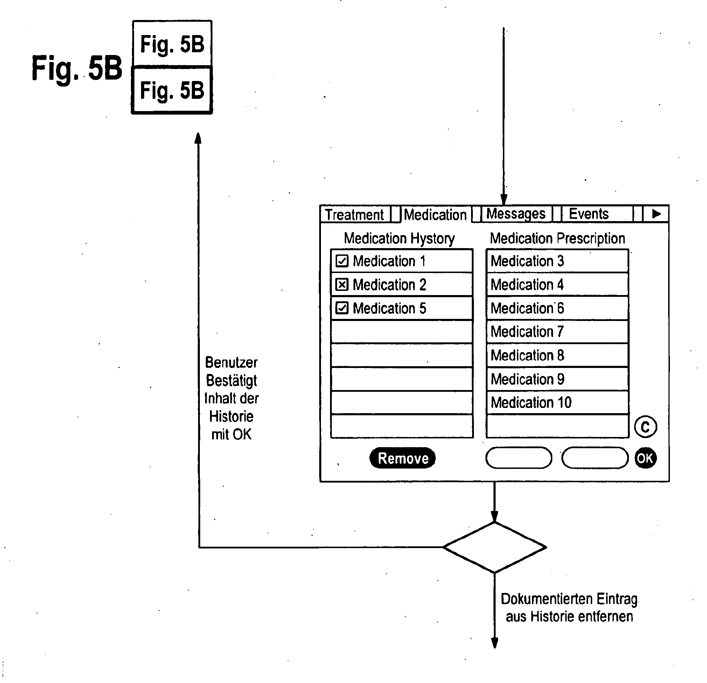

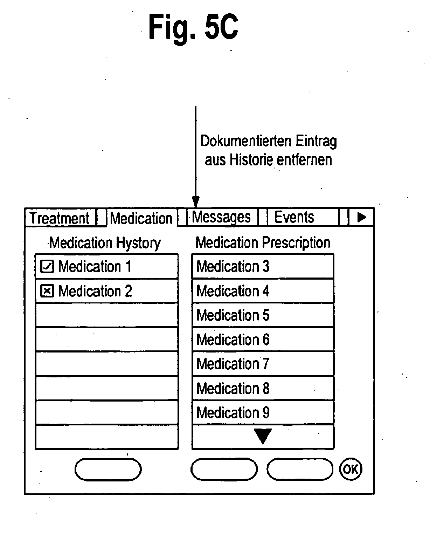

Wenn der Benutzer unter den verschiedenen Gruppen von patientenbezogenen Daten die Seite "Medikamente" auswählt, erscheint eine neue Seite. Die Figuren 5A bis 5C zeigen ein Flussdiagramm zur Erläuterung des logischen Ablaufs der Bedienung nach dem Aufruf der Medikamenten-Seite. Der Benutzer kann innerhalb dieser Gruppe wieder unter einer Mehrzahl von Datensätzen auswählen. Beispielsweise kann der Benutzer unter den vorgegebenen Medikamenten, beispielsweise Medikament 3, Medikament 4, Medikament 5, d.h. aus der Liste der verordneten Medikamente, die von der Vorrichtung zur Bereitstellung von patientenbezogenen Daten geladen wird, das gewünschte Medikament auswählen. Der Schriftzug des ausgewählten Medikaments wird nunmehr grün unterlegt. Nunmehr kann der Benutzer das verordnete Medikament durch Betätigung des Button "Abweisen" bzw. "Annehmen" akzeptieren oder ablehnen. Wenn der Benutzer das ausgewählte Medikament akzeptiert, wird unter der Liste "Medikament History" das ausgewählte Medikament angezeigt und mit einem Haken gekennzeichnet, ansonsten wird das Medikament angezeigt und mit einem Kreuz versehen.

Nach Bestätigung mit "OK" werden alle Einträge in der Liste "Medikament History" zur Dokumentation übernommen und in dem Server 2 unabhängig von der Dialysevorrichtung abgespeichert. Verlässt der Benutzer hingegen den Patientenmodus, gehen alle Eingaben verloren.

Wenn die Seite "Medikament" später wieder aufgerufen wird, werden die patientenbezogenen Datensätze geladen, so dass der Benutzer den Verlauf der Behandlung verfolgen kann. Beispielsweise wird der Benutzer darüber informiert, dass die Medikamente 1 und 5 verabreicht, das Medikament 2 aber nicht verabreicht worden ist.

Neben der Anzeige und Dokumentation von Ereignissen bzw. Aktionen sowie der Verabreichung von Medikamenten stellt der Server dem Benutzer der einzelnen Dialysevorrichtungen auch patientenbezogene Nachrichten zur Verfügung.

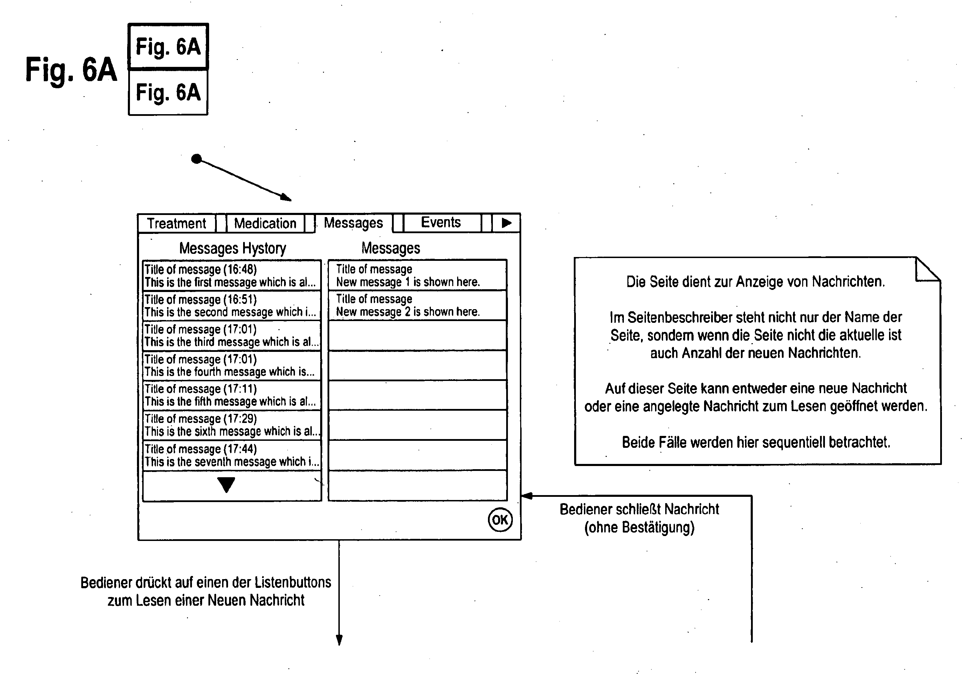

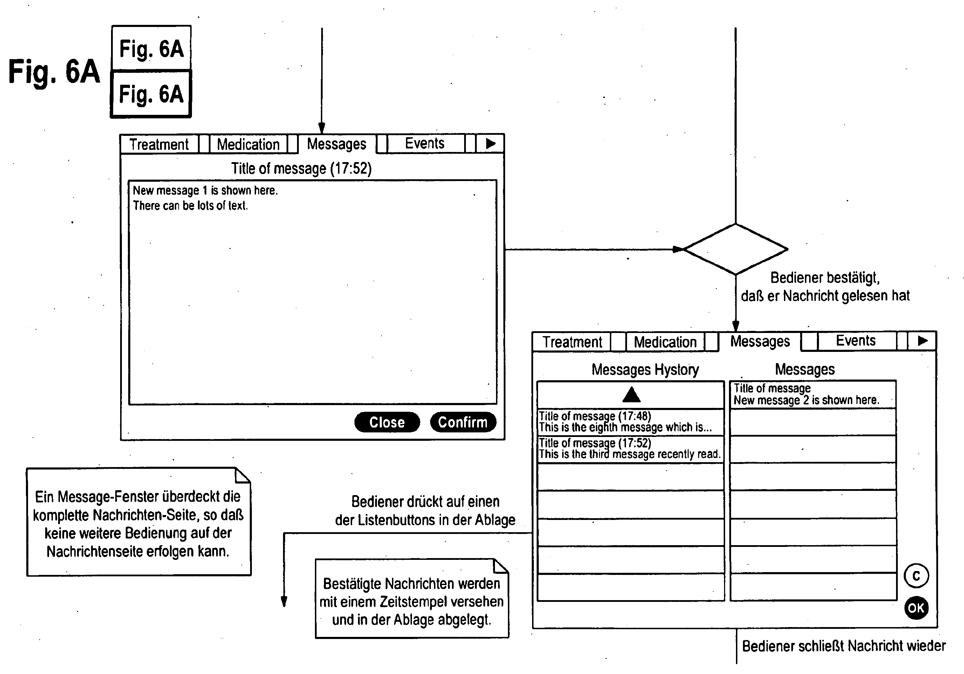

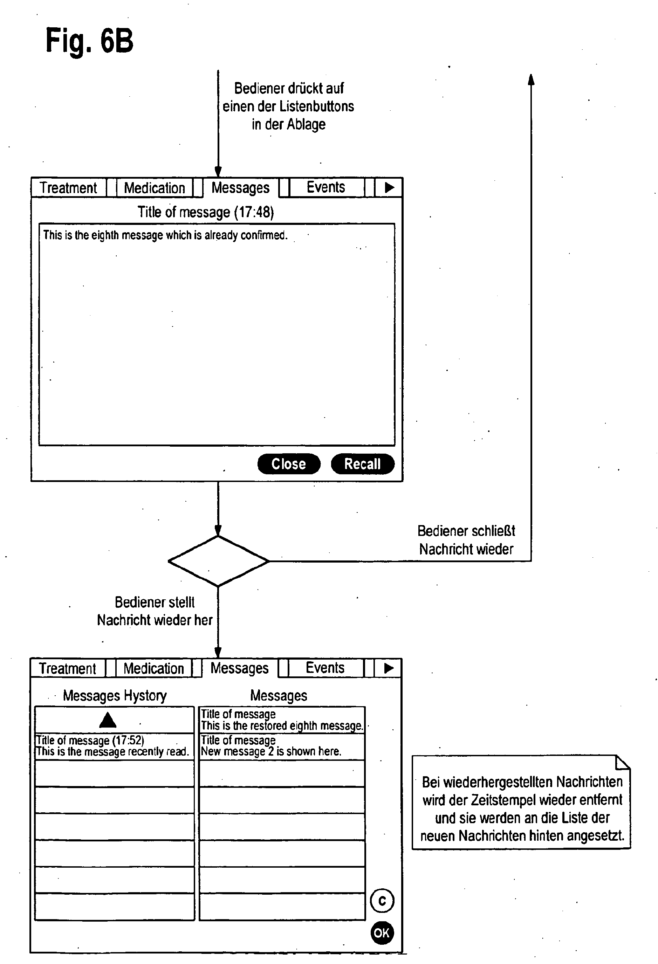

Für jeden Patienten können Nachrichten im Dialysesystem hinterlegt werden. Diese Nachrichten werden mit der Eingabeeinrichtung 2B der Vorrichtung zur Bereitstellung der patientenbezogenen Daten (Server) zentral eingegeben. Beispielsweise können die Nachrichten mit einer Tastatur eingegeben oder auf einem Datenträger eingelesen werden. Die Nachrichten können auch von weiteren nicht dargestellten externen Vorrichtungen zur Verfügung gestellt werden, die mit dem Server kommunizieren. Die Figuren 6A und 6B zeigen ein Flussdiagramm zur Erläuterung des logischen Ablaufs der Bedienung.

Wenn der Behandlungsmodus aufgerufen ist, blinkt beim Vorliegen neuer Nachrichten der Patienten-Button 15 in der Status-Leiste 11. Der Benutzer kann dann das Patientenmenü aktivieren. Er wählt die Seite "Nachrichten". Zum Lesen der Nachrichten wird der entsprechende Button angewählt, so dass sich eine neue Seite öffnet, auf der die Nachrichten unter Angabe der Uhrzeit angezeigt werden. Der Benutzer bestätigt durch Betätigung des Button "Bestätigen", dass er die Nachricht gelesen hat. Andernfalls schließt er die Seite durch Betätigung des Button "Schließen".

Wenn der Benutzer die Nachricht gelesen hat, verschwindet der Titel der Nachricht in der Liste der Nachrichten. Der Titel der Nachricht befindet sich nunmehr in der Liste "Nachrichten History" unter Angabe des Zeitpunktes, zu dem der Benutzer die Nachricht gelesen hat. In der Liste erscheinen auch alle zuvor gelesenen Nachrichten. Der Benutzer kann jetzt weitere Nachrichten, die er noch nicht gelesen hat, der Reihe nach aufrufen und zur Dokumentation unter Angabe der Uhrzeit ablegen.

Um Fehler bei der Dokumentation zu vermeiden, werden alle Inhalte der Seiten mehrmals, mindestens dreimal vom Server abgerufen und in unterschiedlichen Bedienschritten dargestellt, bevor sie in der Datenbank des Servers gespeichert werden können. Die Ausführungsbeispiele zeigen, dass zunächst jedes Element ausgewählt, daraufhin die Auswahl bestätigt und dann schließlich die bestätigte Auswahl in die Liste "History" übernommen werden muss. Durch die mehrfache Eingabe wird die Gefahr von Fehleingaben verringert.

## Patentansprüche

1. Medizinisches Behandlungssystem mit
mindestens einer Vorrichtung zur Durchführung einer medizinischen Behandlung (1, 1', 1"), die eine zentrale Steuereinheit, eine Einrichtung (1B) zur Eingabe und Anzeige von maschinenbezogenen Daten zur Steuerung der Behandlungsvorrichtung und eine Einrichtung (1B) zur Eingabe und Anzeige von patientenbezogenen Daten aufweist, und
einer Vorrichtung (2) zur Bereitstellung von patientenbezogenen Daten, die eine Einrichtung zur Speicherung von patientenbezogenen Daten aufweist,
wobei die mindestens eine Behandlungsvorrichtung und die Vorrichtung zur Bereitstellung von patientenbezogenen Daten derart zusammenwirken, dass mit der Eingabe- / Anzeigeeinrichtung eingegebene Daten mit der Speichereinrichtung abgespeichert und mit der Speichereinrichtung abgespeicherte Daten mit der Eingabe- / Anzeigeeinrichtung angezeigt werden können,
wobei
die Eingabe- / Anzeigeeinrichtung einen als Touchscreen ausgebildeten Bildschirm aufweist, mit dem sowohl maschinenbezogene Daten als auch patientenbezogene Daten eingegeben als auch angezeigt werden können, wobei die patientenbezogenen Daten grafische Elemente definieren, die auf dem Bildschirm dargestellt werden,
die Eingabe- / Anzeigeeinrichtung derart ausgebildet ist, dass in einem Behandlungsmodus maschinenbezogene Datensätze und in einem Patientenmodus patientenbezogene Datensätze angezeigt werden,
und die Eingabe- / Anzeigeeinrichtung derart ausgebildet ist, dass in dem Patientenmodus neben den patientenbezogenen Datensätzen auch maschinenbezogene Datensätze angezeigt werden, wobei in dem Patientenmodus ein patientenbezogener Anzeigebereich (10), der von dem maschinenbezogenen Anzeigebereich verschieden ist, nur zur Anzeige von patientenbezogenen Datensätzen definiert ist, wobei der patientenbezogene Anzeigebereich (10) ein Teil des Bildschirms ist,
**dadurch gekennzeichnet,**
**dass** die Eingabe- / Anzeigeeinrichtung Mittel zum Überprüfen aufweist, die derart ausgebildet sind, dass überprüfbar ist, ob die Darstellung eines von einem patientenbezogenen Datensatz definierten grafischen Element innerhalb der Grenzen des patientenbezogenen Anzeigebereichs (10) ohne Überlappung mit einem bereits dargestellten grafischen Element erfolgen kann, wobei bei einer Überschreitung der Grenzen oder einer Überlappung eine Anzeige der grafischen Elemente verhindert wird, und
**dass** die Eingabe- / Anzeigeeinrichtung derart ausgebildet sind, dass der übrige Teil des Bildschirms bedienbar bleibt, wenn bei einer Überschreitung der Grenzen oder einer Überlappung eine Anzeige der grafischen Elemente in dem patientenbezogenen Anzeigebereich (10) verhindert wird.

2. Medizinisches Behandlungssystem nach Anspruch 1, **dadurch gekennzeichnet, dass** die Eingabe- / Anzeigeeinrichtung derart ausgebildet ist, dass eine Fehlermeldung angezeigt wird, wenn bei einer Überschreitung der Grenzen oder einer Überlappung eine Anzeige der grafischen Elemente in dem patientenbezogenen Anzeigebereich (10) verhindert wird.

3. Medizinisches Behandlungssystem nach Anspruch 1 oder 2, **dadurch gekennzeichnet, dass** die Eingabe- / Anzeigeeinrichtung derart ausgebildet ist, dass in dem Behandlungsmodus ein maschinenbezogener Anzeigebereich nur zur Anzeige von maschinenbezogenen Datensätzen definiert ist.

4. Medizinisches Behandlungssystem nach einem der Ansprüche 1 bis 3, **dadurch gekennzeichnet, dass** die Vorrichtung (2) zur Bereitstellung von patientenbezogenen Daten eine Einrichtung (2B) zur Eingabe von patientenbezogenen Daten aufweist.

5. Medizinisches Behandlungssystem nach einem der Ansprüche 1 bis 4, **dadurch gekennzeichnet, dass** das medizinische Behandlungssystem eine Mehrzahl von Behandlungsvorrichtungen (1, 1', 1") aufweist, wobei die Behandlungsvorrichtungen und die Vorrichtung (2) zur Bereitstellung von patientenbezogenen Daten zu einem Netzwerk (3) miteinander verbunden sind.

6. Medizinisches Behandlungssystem nach einem der Ansprüche 1 bis 5, **dadurch gekennzeichnet, dass** die Behandlungsvorrichtung (1, 1', 1") eine extrakorporale Blutbehandlungsvorrichtung ist.

## Claims

1. Medical treatment system with
at least one device for carrying out medical treatment (1, 1', 1"), which comprises a central control unit, an apparatus (1B) for input and display of machine-related data for control of the treatment device and an apparatus (1B) for input and display of patient-related data, and
a device (2) for provision of patient-related data, which has an apparatus for storage of the patient-related data,
wherein the at least one treatment device and the device for provision of patient-related data interact so that data input with the input/display apparatus can be stored with the storage apparatus and the data stored with the storage apparatus can be displayed with the input/display apparatus,
wherein the input/display apparatus has a screen configured as touch screen, with which both machine-related data and patient-related data can be input and also displayed, wherein the patient-related data define graphic elements which are shown on the screen,
the input/display apparatus is configured so that, in a treatment mode, machine-related data records are displayed and, in a patient mode, patient-related data records
and the input/display apparatus is configured so that machine-related data records are displayed in the patient mode as well as the patient-related data records, wherein, in the patient mode, a patient-related display area (10) is defined that is different from the machine-related display area, only for display of patient-related data records, wherein the patient-related display area (10) is part of the screen,
**characterised in that**
the input/display apparatus has means for making checks, which are configured so that it is possible to check whether the presentation of a patient-related data record defined graphic element can be made within the limits of a patient-related display area (10) without overlapping a graphic element that is already displayed, such that, when the limits are exceeded or there is an overlap, display of the graphic elements is prevented, and
that the input/display apparatus is configured so that the remaining part of the screen continues to be accessible if a display of graphic elements in the patient-related display area (10) is prevented when limits are exceeded or there is overlapping.

2. Medical treatment system in accordance with claim 1, **characterised in that** the input/display apparatus is configured so that an error report is displayed when a display of graphic elements in the patient-related display area (10) is prevented when limits are exceeded or there is an overlap.

3. Medical treatment system in accordance with claim 1 or 2, **characterised in that** the input/display apparatus is configured so that, in treatment mode, a machine-related display area is only defined for display of machine-related data records.

4. Medical treatment system in accordance with claims 1 to 3, **characterised in that** the device (2), for provision of patient-related data comprises an apparatus (2B) for input of patient-related data.

5. Medical treatment system in accordance with one of the claims 1 to 4, **characterised in that** the medical treatment system comprises a plurality of treatment devices (1, 1', 1"), wherein the treatment device and the device (2) for provision of patient-related data are connected with one another to a network (3).

6. Medical treatment system in accordance with one of the claims 1 to 5, **characterised in that** the treatment device (1, 1', 1 ") is an extracorporeal blood treatment device.

## Revendications

1. Système de traitement médical comportant
au moins un dispositif de réalisation d'un traitement médical (1, 1', 1") qui présente une unité de commande centrale, une installation (1B) d'entrée et d'affichage de données relatives à la machine pour la commande du dispositif de traitement et une installation (1B) d'entrée et d'affichage de données relatives au patient, et
un dispositif (2) de mise à disposition de données relatives au patient, qui présente une installation de mémorisation des données relatives au patient,
dans lequel au moins le dispositif de traitement et le dispositif de mise à disposition des données relatives au patient coopèrent de telle sorte que des données entrées avec l'installation d'entrée/d'affichage soient mémorisées avec l'installation de mémorisation et que des données mémorisées avec l'installation de mémorisation puissent être affichées avec l'installation d'entrée/d'affichage,
dans lequel
l'installation d'entrée/d'affichage présente un écran constitué comme un écran tactile avec lequel à la fois des données relatives à la machine et des données relatives au patient peuvent être entrées et également affichées, les données relatives au patient définissant des éléments graphiques qui sont représentés sur l'écran,
l'installation d'entrée/d'affichage étant conçue de telle sorte que, dans un mode de traitement, des jeux de données relatives à la machine et, dans un mode patient, des jeux de données relatives au patient soient affichés
et l'installation d'entrée/ d'affichage étant conçue de telle sorte que, dans le mode patient, outre les jeux de données relatives au patient, des jeux de données relatives à la machine soient aussi affichés, une zone d'affichage (10) relative au patient, qui est différente de la zone d'affichage relative à la machine, étant définie dans le mode patient uniquement pour l'affichage de jeux de données relatives au patient, la zone d'affichage (10) relative au patient étant une partie de l'écran,
**caractérisé en ce que**
l'installation d'entrée/d'affichage présente des moyens de vérification qui sont conçus de telle sorte que l'on puisse vérifier si la représentation d'un jeu de données relatives au patient par un élément graphique défini peut s'effectuer dans les limites de la zone d'affichage (10) relative au patient sans chevaucher un élément graphique déjà affiché, en cas de dépassement des limites ou de chevauchement, un affichage des éléments graphiques étant empêché, et
**en ce que** l'installation d'entrée/d'affichage est conçue de telle sorte que la partie restante de l'écran reste utilisable lorsque, dans le cas d'un dépassement des limites ou d'un chevauchement, un affichage des éléments graphiques dans la zone d'affichage (10) relative au patient est empêché.

2. Système de traitement médical selon la revendication 1, **caractérisé en ce que** l'installation d'entrée/d'affichage est conçue de telle sorte qu'un message d'erreur s'affiche lorsque, dans le cas d'un dépassement des limites ou d'un chevauchement, un affichage des éléments graphiques dans la zone d'affichage (10) relative au patient est empêché.

3. Système de traitement médical selon la revendication 1 ou 2, **caractérisé en ce que** l'installation d'entrée/d'affichage est conçue de telle sorte que, dans le mode de traitement, une zone d'affichage relative à la machine est définie uniquement pour l'affichage de jeux de données relatives à la machine.

4. Système de traitement médical selon l'une des revendications 1 à 3, **caractérisé en ce que** le dispositif (2) de mise à disposition de données relatives au patient présente une installation (2B) pour l'entrée de données relatives au patient.

5. Système de traitement médical selon l'une des revendications 1 à 4, **caractérisé en ce que** le système de traitement médical présente une multiplicité de dispositifs de traitement (1, 1', 1"), les dispositifs de traitement et le dispositif (2) de mise à disposition de données relatives au patient étant reliés les uns aux autres en un réseau (3).

6. Système de traitement médical selon l'une des revendications 1 à 5, **caractérisé en ce que** le dispositif de traitement (1, 1', 1") est un dispositif de traitement sanguin extracorporel.
